Europäisches Patentamt

European Patent Office

Office européen des brevets

(19)

(11) Veröffentlichungsnummer: **0 184 785 B1**

## (12) EUROPÄISCHE PATENTSCHRIFT

(45) Veröffentlichungstag der Patentschrift: **11.09.91**

(21) Anmeldenummer: **85115512.7**

(22) Anmeldetag: **06.12.85**

(51) Int. Cl.⁵: **C08F 220/18**, A61K 7/13,
A61K 7/135, C01B 15/01,
//(C08F220/18,220:06,220:06,
246:00)

(54) **Acrylatdispersion und deren Verwendung zur Verdickung von Wasserstoffperoxid-Zubereitungen.**

(30) Priorität: **14.12.84 DE 3445549**

(43) Veröffentlichungstag der Anmeldung:
**18.06.86 Patentblatt 86/25**

(45) Bekanntmachung des Hinweises auf die
Patenterteilung:
**11.09.91 Patentblatt 91/37**

(84) Benannte Vertragsstaaten:
**AT BE CH DE FR GB IT LI NL SE**

(56) Entgegenhaltungen:
**GB-A- 1 062 338**

(73) Patentinhaber: **Henkel Kommanditgesellschaft
auf Aktien
Postfach 1100 Henkelstrasse 67
W-4000 Düsseldorf-Holthausen(DE)**

(72) Erfinder: **Schieferstein, Ludwig, Dr.
Am Hang 15
W-4030 Ratingen 1(DE)**
Erfinder: **Hollenberg, Detlef, Dr.
Hugo-Wolf-Strasse 15
W-4010 Hilden(DE)**
Erfinder: **Maak, Norbert, Dr.
Im Jagdfeld 41 a
W-4040 Neuss(DE)**

(74) Vertreter: **von Kreisler, Alek, Dipl.-Chem. et al
Patentanwälte Von Kreisler-Selting-Werner,
Deichmannhaus am Hauptbahnhof
W-5000 Köln 1(DE)**

## Beschreibung

Gegenstand der Erfindung ist eine Wasserstoffperoxidzubereitung, enthaltend eine wäßrige Copolymerisat-Dispersion aus Ethylacrylat, Methacrylsäure, Acrylsäure und einem mehrfach ungesättigten copolymerisierbaren Monomeren mit einem Feststoffgehalt von 5 - 30 Gew.-% bezogen auf die Dispersion, die sich besonders gut zur Verdickung wäßriger Systeme, insbesondere von Wasserstoffperoxid-Zubereitungen eignet, wie sie als Entwickler-Zubereitungen für Oxidationshaarfärbemittel und für Haarbleichmittel verwendet werden.

Für die Verdickung wäßriger Systeme ist die Verwendung von alkalilöslichen Acrylatdispersionen seit langem bekannt. Solche Dispersionen von Copolymerisaten aus wenigstens 10 Gew.-% Acrylsäure-Niedrigalkylester, 25 - 70 Gew.-% Methacrylsäure und ggf. 0 - 40 Gew.-% eines weiteren Comonomers mit einem Feststoffgehalt von 25 - 50 Gew.-% werden z. B. in GB-PS-870 994 beschrieben. Aus DE-AS-1.164.095 sind Mischpolymerisate aus 50 - 75 Gew.-% Ethylacrylat, 25 - 35 Gew.-% Acrylsäure und 0 - 25 Gew.-% anderer Comonomerer bekannt. In den genannten Druckschriften wird auch die Möglichkeit, durch Zugabe von vernetzend wirkenden, mehrfach ungesättigten copolymerisierbaren Comonomeren die Molekulargewichte der Polymeren und damit deren Verdickungswirkung zu erhöhen, angeführt. In GB-PS-870 994 wird angegeben, daß ein Gehalt von 0,1 - 0,8 Gew.-% und besonders von 0,15 - 0,3 Gew.-% der vernetzenden Comonomeren zu besonders günstigen Polymerisaten führt.

Aus GB-A-1 062 338 ist eine wäßrige Dispersion eines Copolymerisats aus Ethylacrylat, Methacrylsäure und beispielsweise Ethylenglykoldimethacrylat bekannt. Derartige Emulsionen weisen Feststoffgehalte von 15 bis 20 % auf. Aus den Ansprüchen der GB-A-1 062 338 geht hervor, daß das Vernetzungsmittel bevorzugt ein Diester der Acryl- bzw. Methacrylsäure, wie z.B. Ethylenglykoldimethacrylat und die copolymerisierbare Säure Acryl- oder Methacrylsäure ist.

Die aus den genannten Druckschriften bekannten Copolymerisat-Dispersionen erfüllen jedoch nicht die hohen Anforderungen, die an wirkungsvolle Verdickungsmittel gestellt werden: Die neutralisierten Dispersionen neigen oft zu starkem Fadenziehen, was sie für Anwendungen in der Kosmetik wegen des damit verbundenen schleimigen Hautgefühls unbrauchbar macht. Andere Dispersionen weisen nur ein mäßiges Verdickungsvermögen auf, oder die Viskosität der verdickten Zubereitungen ist extrem störungsanfällig, d. h. sie wird durch Rezepturbestandteile wie z. B. Elektrolyte entweder stark erniedrigt oder in den Gelbereich angehoben. Ungünstig ist auch eine zu starke Abhängigkeit der Viskosität von der Konzentration des Verdickers. Besonders hohe Anforderungen werden an alkalilösliche Acrylat-Copolymerdispersionen gestellt, die zur Verdickung von Wasserstoffperoxid-Zubereitungen eingesetzt werden, wie sie als Entwickler-Zubereitungen für Oxidationshaarfärbemittel oder für Haarbleichmittel gebräuchlich sind. Diese, schwach sauer eingestellten Zubereitungen werden kurz vor der Anwendung mit der alkalischen Färbecreme zu einem alkalischen Färbemittel vermischt. Dadurch wird die Konsistenz der Creme zerstört. Die in der Entwicklerzubereitung enthaltene Verdickerdispersion soll daher in dem nach der Vereinigung der beiden Komponenten alkalischen Färbemittel eine für die Anwendung auf den Haaren geeignete Viskosität aufbauen. Während sich die Rezepturen von verschiedenen Oxidationshaarfärbepräparaten teilweise erheblich von einander unterscheiden, soll die Entwicklerzubereitung bei stets gleicher Dosierung auch stets die gleiche, für die Anwendung günstige Konsistenz der Mischung aus Färbegrundlage und Entwicklerzubereitung sicherstellen. Dies ist nur möglich, wenn das für den Konsistenzaufbau verantwortliche Verdickungsmittel besonders wirksam und gegenüber störenden Einflüssen unempfindlich ist.

Ähnliches gilt für Haarbleichmittel. Diese werden ebenfalls als 2-Komponentenprodukte formuliert, bestehend aus einer schwach sauer eingestellten Wasserstoffperoxid-Zubereitung und einer alkalisch eingestellten Blondiercreme, die vor der Anwendung miteinander vermischt werden. Die Wasserstoffperoxid-Zubereitung soll dabei eine Verdickerdispersion enthalten, die dem nach der Vereinigung der Komponenten alkalischer Blondiermittel eine für die Anwendung auf dem Haar geeignete Viskosität verleiht.

Es wurde gefunden, daß eine Wasserstoffperoxidzubereitung, enthaltend eine wäßrige Dispersion eines Copolymerisats mit einem Feststoffgehalt von 5 - 30 Gew.-% bezogen auf die Dispersion, aus

| 50 - 60 Gew.-% | Ethylacrylat |
| 30 - 40 Gew.-% | Methacrylsäure |
| 5 - 15 Gew.-% | Acrylsäure und |
| 0,02 - 0,04 Gew.-% | eines mehrfach ungesättigten, copolymerisierbaren Monomeren |

besteht, eine besonders hohe Verdickungswirkung für wäßrige Lösungen nach Neutralisation der freien Carboxylgruppen mit wasserlöslichen Alkalien aufweist, daß die verdickten wäßrigen Lösungen frei von Trübungen sind und daß die Viskosität und die Klarlöslichkeit relativ stabil gegen den Einfluß von gelösten Salzen ist.

2

EP 0 184 785 B1

Insbesondere bevorzugt ist die Anwesenheit eines zweifach ungesättigten, copolymerisierbaren Monomeren.

Aufgrund der genannten Eigenschaften eignet sich die erfindungsgemäße Wasserstoffperoxid zubereitung, um diesen nach Neutralisation, insbesondere nach Erhöhung des pH-Wertes auf Werte oberhalb +8 eine erhöhte Viskosität zu verleihen.

Die Herstellung der Copolymerisat-Dispersion erfolgt nach dem an sich bekannten Verfahren unter Verwendung eines geeigneten, an sich bekannten Emulgator- und Stabilisatorsystems und eines an sich bekannten Startersystems. Als Emulgator und Stabilisator sind z. B. Nonylphenoxypolyethoxysulfosuccinate und/oder Alkylpolyethoxysulfate in Form ihrer Natrium- und/oder Ammoniumsalze geeignet, wie sie in DE-OS-2 107 651 beschrieben sind. Die Anwendungsmenge dieser Emulgatoren ist so hoch zu wählen, daß eine ausreichende Feinteiligkeit und Stabilität der Dispersion erreicht wird. Mengen von 1 - 4 Gew.-% solcher Emulgatoren bezogen auf die Dispersion sind in der Regel ausreichend. Als radikalischer Starter wird bevorzugt Kalium- oder Ammoniumperoxidisulfat verwendet, es sind jedoch auch andere bekannte Radikalbildner wie z. B. tert.-Butylhydroperoxid geeignet. Bei Verwendung von Kalium- oder Ammoniumperoxidisulfat sind Mengen von 0,002 - 0,01 Gew.-% der Dispersion ausreichend.

Die mehrfach ungesättigten copolymerisierbaren Monomeren dienen dazu, das Molekulargewicht des Copolymerisats durch Vernetzung gezielt zu erhöhen. Als copolymerisierbare, zweifach ungesättigte Monomere eignen sich besonders gut Ester der Acryl- und Methacrylsäure wie z. B. Ethylenglycoldimethacrylat, Trimethylolpropan-trimethacrylat, Polyethylenglykol (400)-dimethacrylat, Allylmethacrylat, 2-Ethylhexan-1.3-dimethacrylat. Die Menge der zur Vernetzung dienenden mehrfach ungesättigten copolymerisierbaren Monomeren ist von großem Einfluß auf die Eigenschaften des Copolymerisats, da ein zu geringer Anteil des Vernetzungsmittels keine ausreichenden Verdickungseigenschaften und eine starke Neigung zum Fädenziehen, ein zu hoher Anteil des Vernetzungsmittels das Auftreten von Trübungen oder Gelbildung im neutralisierten Polymerisat bewirkt. Besonders geeignete Polymerdispersionen werden erhalten, wenn das Copolymerisat aus

| | |
|---|---|
| 55 Gew.-% | Ethylacrylat |
| 35 Gew.-% | Methacrylsäure |
| 10 Gew.-% | Acrylsäure und |
| 0,025 Gew.-% | Ethylenglycoldimethacrylat besteht. |

Als Verfahren zur Herstellung der Copolymerisat-Dispersionen eignet sich besonders das sogenannte Monomeren-Zulaufverfahren. Dabei wird eine wäßrige Lösung, die etwa ein Drittel des Starters und etwa 40 Gew.-% der Emulgatoren enthält, vorgelegt und über der Lösung eine Inertgasatmosphäre (Kohlendioxid oder Stickstoff) erzeugt. In die auf ca. 70 - 80 °C erwärmte Vorlage wird zunächst ein Teil des Monomerengemisches unter Rühren eingetragen und nach Reaktionsstart der Rest des Monomerengemisches und die Lösung der restlichen Emulgatormenge und ein zweites Drittel des Starters gleichzeitig unter Abführung der Polymerisationswärme nach und nach eingetragen. Nach Abklingen der Polymerisations-Wärmeentwicklung empfiehlt es sich, durch Nachdosieren einer wäßrigen Lösung des letzten Drittels der Startermenge einen weitgehenden Umsatz der Monomeren sicherzustellen.

Die Copolymerisat-Dispersionen lassen sich mit einem Feststoffgehalt bis zu 50 Gew.-% herstellen. Es hat sich jedoch gezeigt, daß bei Einhaltung eines Feststoffgehaltes von 5 - 30 Gew.-%, bevorzugt von 20 - 30 Gew.-% sehr niedrige Anteile an grobteiligen Koagulaten entstehen, die nach der Polymerisation durch Filtration entfernt werden müssen.

Die Copolymerisat-Dispersionen sind in wäßriger Lösung dünnflüssig und stabil. Sie bleiben auch in schwach sauren wäßrigen Lösungen mit pH-Werten von 2 - 5 stabil und dünnflüssig. In dem Maße, wie der pH-Wert der verdünnten wäßrigen Lösungen durch wäßrige Basen, z. B. durch Ammoniaklösung, Alkalihydroxydlösungen (z. B. Natronlauge, Kalilauge), Alkanolamine (z. B. Mono-, Di- oder Triethanolamin) angehoben und die Carboxylgruppen des Copolymerisats neutralisiert, d. h. in die Salzform überführt werden, beginnen die Polymerketten sich zu entknäueln und die Viskosität der Lösungen zu erhöhen. Bei einem pH-Wert von 8 ist die völlige Ionisation der Carboxylgruppen und die Viskositätsentwicklung abgeschlossen. Die Viskosität der verdickten Lösungen nimmt aber auch bei weiterem Alkalizusatz nur wenig ab und ist auch gegenüber dem Zusatz von wasserlöslichen Salzen weitgehend unempfindlich.

Daher eignen sich die Copolymer-Dispersionen ganz besonders für die Herstellung von Wasserstoffperoxidzubereitungen zur Verwendung als Entwickler für Oxidationshaarfärbemittel oder für Haarbleichmittel. Solche Entwicklerdispersionen sind gekennzeichnet durch einen Gehalt von

| | |
|---|---|
| 1 - 20 Gew.-% | Wasserstoffperoxid und |
| 1 - 5 Gew.-% | (Feststoff) eines Copolymerisats |

einer Copolymerisat-Dispersion und stellen eine spezielle Ausführungsform der Erfindung dar. Neben den kennzeichnenden Bestandteilen enthalten solche Entwicklerdispersionen noch grenzflächenaktive Substan-

3

zen, bevorzugt anionische oder nichtionische synthetische Tenside in Mengen von 0,2 - 5,0 Gew.-%. Als anionische Tenside können Fettalkoholsulfate oder Fettalkoholpolyglycolethersulfate in Form der Natrium, Kalium, Ammonium oder Alkanolammoniumsalze enthalten sein. Zur Stabilisierung des Wasserstoffperoxids können z. B. Dipicolinsäure, Chinolinsäure, Polyphosphate oder die aus DE-PS-1 107 207 bekannten Acylierungsprodukte der phosphorigen Säure, z. B. die 1-Hydroxyethan-1.1-diphosphonsäure in einer Menge von 0,05 - 1,5 Gew.-% enthalten sein. Zur Einstellung eines schwach sauren pH-Wertes von ca. 2 - 5 wird bevorzugt saures Natriumpyrophosphat ($Na_2H_2P_2O_7$) verwendet. Zur Verbesserung der kosmetischen Eigenschaften können außerdem Duftstoffe und wasserlösliche Proteinderivate zugegeben werden.

Die Entwicklerdispersionen werden unmittelbar vor der Anwendung mit einer deutlich alkalisch eingestellten Färbegrundlage oder Blondiergrundlage vermischt, wobei die Mischung einen schwach alkalischen pH-Wert von ca. 8 - 10 aufweist. Je nach Wasserstoffperoxid-Konzentration und gewünschter Wirkung beträgt das Mischungsverhältnis von Entwicklerdispersion zu Färbe- oder Blondierungsgrundlage 2 : 1 bis 1 : 2 (Gewichtsteile).

Die Färbe- und Blondiergrundlagen sind meist Emulsionsgrundlagen, d. h. cremeförmige Emulsionen vom Typ Öl-in-Wasser. Als Fettphase sind bevorzugt Fettalkohole enthalten, die mit anionischen Emulgatoren, bevorzugt Fettalkoholsulfat, Fettalkoholpolyglycolethersulfat oder auch mit nichtionogenen Emulgatoren, z. B. Anlagerungsprodukten von Ethylenoxid an Fettalkohole, Fettsäuren, Fettsäurepartialglyceriden, Sorbitanfettsäureestern oder Fettsäurealkanolamiden in eine cremeförmige Emulsion überführt werden. Diese Emulsionsgrundlagen enthalten in der Regel 7 - 15 Gew.-% Fettalkohol, der mit Hilfe von 4 - 10 Gew.-% der genannten Emulgatoren (bezogen auf die gesamte Emulsion) emulgiert ist. In diese Emulsionsgrundlagen sind bei den Färbegrundlagen die Oxidationsfarbstoffvorprodukte eingearbeitet. Die Blondier-Cremegrundlagen enthalten in der Regel keine Oxidationsfarbstoffvorprodukte, können aber auch kleine Mengen an Oxidationsfarbstoffvorprodukten zur Mattierung enthalten, um einen bei der Blondierung brauner Haare leicht auftretenden Gelb- oder Rotschimmer zu kompensieren. Darüber hinaus enthalten Färbe- und Blondiergrundlagen Basen, bevorzugt $NH_3$-Lösung und Puffersalze (Ammoniumsalze), um einen alkalischen pH-Wert von ca. 9 - 11 zu stabilisieren, sowie Duftstoffe und haarkosmetische Wirkstoffe wie z. B. Proteinderivate und kationische Polymere in kleineren Mengen.

Bei der Vermischung der Färbe- oder Blondiergrundlagen mit den Entwicklerdispersionen ($H_2O_2$-Zubereitungen) kommt es zu einem weitgehenden Zusammenbruch der Konsistenz der Cremegrundlage. Durch den in der Mischung sich einstellenden alkalischen pH-Wert kommt es dabei aber zum Aufbau der durch die erfindungsgemäße Copolymer-Dispersion verursachten Viskosität. Auf diese Weise werden Färbezubereitungen oder Bleichzubereitungen erhalten, die sich gut mit einem Pinsel oder aus einem mit einer Applikationsdüse versehenen Applikationsgefäß auf das Haar aufbringen lassen. Die bekannten Probleme, die bei zu niedriger Konsistenz solcher Färbe- oder Bleichzubereitungen auftreten, insbesondere das Verlaufen der Färbe- oder Bleichzubereitung am Haar entlang und die Färbung und Bleichung nicht zu behandelnder Haarpartien werden auf diese Weise erfolgreich vermieden. Darüber hinaus wird ein brillantes Färbeergebnis erhalten.

Die folgenden Beispiele sollen den Gegenstand der Erfindung näher erläutern.

**Beispiele**

1. Eine Lösung aus
| | |
|---|---|
| 62,5 Gew.-Teilen | Wasser (vollentsalzt) |
| 0,76 Gew.-Teilen | Nonylphenoxypolyethoxy(9 EO)-sulfosuccinat, Dinatriumsalz |
| 0,76 Gew.-Teilen | Natrium-alkyl($C_{12-18}$)polyethoxy(10 EO)sulfat |
| 0,002 Gew.-Teilen | Ammoniumperoxydisulfat |

wurde in das Reaktionsgefäß gegeben. Unter Rühren auf niedriger Rührstufe wurde 1 Gew.-Teil Trockeneis ($CO_2$) zugesetzt. Das entstehende $CO_2$-Gas stellte in allen Teilen der Apparatur eine sauerstofffreie Atmosphäre her. Ein weiteres Gew.-Teil Trockeneis wurde, nach dem die Dosierlösungen in die Tropftrichter gefüllt waren, auf diese verteilt, so daß auch dort eine sauerstofffreie Atmosphäre entstand.

Die Vorlage wurde auf 75 °C aufgeheizt und 20 Gew.-% der Zulauflösung I aus
| | |
|---|---|
| 15,86 Gew.-Teilen | Ethylacrylat |
| 10,00 Gew.-Teilen | Methacrylsäure |
| 2,86 Gew.-Teilen | Acrylsäure |
| 0,007 Gew.-Teilen | Ethylenglycoldimethacrylat und |
| 0,29 Gew.-Teilen | Essigsäureanhydrid |

zugegeben. Nach 5 - 10 Minuten zeigte ein Temperaturaufstieg auf 78 °C den Reaktionsstart an.

Innerhalb von 25 - 30 Minuten wurden nun gleichzeitig die Zulauflösung II aus

| | |
|---|---|
| 4,58 Gew.-Teilen | Wasser |
| 1,14 Gew.-Teilen | Nonylphenoxypolyethoxy(9 EO)-sulfosuccinat, Dinatriumsalz |
| 1,14 Gew.-Teilen | Natriumalkyl($C_{12-18}$)polyethoxy(10 EO)-sulfat |
| 0,002 Gew.-Teilen | Ammoniumperoxydisulfat |

und der Rest der Zuflauflösung I gleichmäßig zugetropft. Dabei wurde die Reaktionstemperatur durch Kühlung auf 80 - 85 °C gehalten. Nach beendetem Zulauf wurde so lange nachgerührt, bis nach ca. 20 - 30 Minuten die Wärmeentwicklung beendet war. Dann wurde die Lösung aus

| | |
|---|---|
| 0,1 Gew.-Teilen | Wasser und |
| 0,002 Gew.-Teilen | Ammoniumperoxydisulfat |

zugegeben und weitere 60 Minuten bei 75 °C gerührt. Nach dem Abkühlen auf 20 °C wurde die Dispersion durch einen Kunststoffsiebbeutel mit einer Maschenweite von 250 µ abgelassen.

Die erhaltene Dispersion hatte einen Feststoffgehalt von 28,5 Gew.-%. Die Viskosität einer auf 1 Gew.-% Feststoffgehalt verdünnten und mit NH$_3$-Lösung auf pH = 8 eingestellten Lösung bei 20 °C betrug 2500 mPa.s (gemessen mit dem Brookfield-Rotationsviskosimeter bei 20 UpM).

2. Nach dem gleichen Verfahren wie in Beispiel 1 wurde eine zweite Dispersion hergestellt, wobei die Zulauflösung I bestand aus

| | |
|---|---|
| 15,86 Gew.-Teilen | Ethylacrylat |
| 11,40 Gew.-Teilen | Methacrylsäure |
| 1,45 Gew.-Teilen | Acrylsäure |
| 0,007 Gew.-Teilen | Ethylenglycoldimethacrylat |
| 0,29 Gew.-Teilen | Essigsäureanhydrid |

Die erhaltene Dispersion hatte nach Verdünnung auf 1 Gew.-% Feststoffgehalt und pH-Einstellung (mit NH$_3$-Lösung) auf pH = 8 eine Viskosität von 2200 mPa.s (20 °C).

3. Anwendungsbeispiele

| Färbe/Blondiergrundlage | Tönungs-grundlage | Färbe-creme | Blondier-creme |
|---|---|---|---|
| Fettalkohol $C_{12}$-$C_{14}$ + 2 EO-sulfat Na-Salz (28 %ig) | 26,5 Gew.-% | 26,5 Gew.-% | 26,5 Gew.-% |
| Fettalkoholgemisch $C_{12}$-$C_{18}$ | 10,5 " | 10,0 " | 12,0 " |
| Oxidationsfarbstoffvorprodukte | 0,15 " | 1,5 " | - |
| Proteinhydrolysat | 0,10 " | 0,10 " | 0,15 " |
| Parfümöl | 0,20 " | 0,2 " | 0,4 " |
| $Na_2SO_3$ | 1,0 " | 1,0 " | - |
| $(NH_4)_2SO_4$ | 0,2 " | 1,2 " | 1,0 " |
| $NH_4Cl$ | 0,45 " | - | - |
| Wasser, ($NH_3$-Lsg. bis pH = 9,5) | ad 100 " | ad 100 " | ad 100 " |

| | für Tönungs-grundlage | für Färbe-creme | für Blondier-creme |
|---|---|---|---|
| Entwicklerdispersion | | | |
| Fettalkohol $C_{12}$-$C_{14}$ + 2 EO-sulfat Na-Salz (28%ig) | 2,0 Gew.-% | 2,0 Gew.-% | 2,0 Gew.-% |
| Proteinhydrolysat | 0,5 " | 0,5 " | 0,15 " |
| 1-Hydroxyethan-1.1-diphosphonsäure | 0,2 " | 0,2 " | 1,7 " |
| $H_2O_2$ (35%ige Lösung) | 8,5 " | 17 " | 34 " |
| Copolymerisat-Dispersion Beispiel 1 | 8 " | 10 " | 6 " |
| Wasser, ($Na_2H_2P_2O_7$ bis pH = 3,8) | ad 100" | ad 100 " | ad 100 " |
| Mischungsverhältnis von Färbe/Blondiergrundlage zu Entwicklerdispersion | 2 : 1 | 1 : 1 | 1 : 1 |
| Viskosität (20 °C) bei D = 6 $sec^{-1}$ (nach $\eta = \frac{\tau}{D}$) | 3950 mPa.s (20 °C) | 2866 mPa.s (20 °C) | 4266 mPa.s (20 °C) |

## Patentansprüche

**Patentansprüche für folgende Vertragsstaaten : BE, CH, DE, FR, GB, IT, LI, NL, SE**

1. Wasserstoffperoxidzubereitung enthaltend eine wäßrige Dispersion eines Copolymerisats mit einem Feststoffgehalt von 5 - 30 Gew.-% bezogen auf die Dispersion aus Dispersion, aus

    | | |
    |---|---|
    | 50 - 60 Gew.-% | Ethylacrylat |
    | 30 - 40 Gew.-% | Methacrylsäure |
    | 5 - 15 Gew.-% | Acrylsäure und |
    | 0,02 - 0,04 Gew.-% | eines mehrfach ungesättigten, copolymerisierbaren Monomeren besteht. |

2. Wasserstoffperoxidzubereitung nach Anspruch 1, dadurch gekennzeichnet, daß das Copolymerisat 0,02 bis 0,04 Gew.-% eines zweifach ungesättigten, copolymerisierbaren Monomeren enthält.

3. Wasserstoffperoxidzubereitung nach Anspruch 1 oder 2, dadurch gekennzeichnet, daß das Copolymerisat aus

7

| 55 Gew.-% | Ethylacrylat |
|---|---|
| 35 Gew.-% | Methycrylsäure |
| 10 Gew.-% | Acrylsäure und |
| 0,025 Gew.-% | Ethylenglycoldimethacrylat besteht. |

4. Wasserstoffperoxidzubereitung nach Ansprüchen 1 bis 3, gekennzeichnet durch einen pH-Wert von 2 - 5 und einen Gehalt von

| 1 - 20 Gew.-% | Wasserstoffperoxid und |
|---|---|
| 1 - 5 Gew.-% | (Feststoff) eines Copolymerisats einer Dispersion gemäß Ansprüchen 1 bis 3. |

5. Wasserstoffperoxidzubereitung nach Ansprüchen 1 bis 4, gekennzeichnet durch einen pH-Wert von mehr als 8.

6. Verwendung der Wasserstoffperoxidzubereitung nach Anspruch 5 als Entwickler für Oxidationsfärbemittel oder Haarbleichmittel.

**Patentansprüche für folgenden Vertragsstaat : AT**

1. Verfahren zur Herstellung einer Wasserstoffperoxidzubereitung enthaltend eine wäßrige Dispersion eines Copolymerisats mit einem Feststoffgehalt von 5 - 30 Gew.-% bezogen auf die Dispersion, aus

| 50 - 60 Gew.-% | Ethylacrylat |
|---|---|
| 30 - 40 Gew.-% | Methacrylsäure |
| 5 - 15 Gew.-% | Acrylsäure und |
| 0,02 - 0,04 Gew.-% | eines mehrfach ungesättigten, copolymerisierbaren Monomeren besteht, |

durch das Monomeren-Zulaufverfahren, wobei man eine wäßrige Lösung, die etwa ein Drittel des Starters und etwa 40 Gew.-% der Emulgatoren enthält, vorlegt und über der Lösung eine Inertgasatmosphäre erzeugt, in die auf 70 - 80 °C erwärmte Vorlage zunächst einen Teil des Monomerengemisches unter Rühren einträgt und nach dem Reaktionsstart den Rest des Monomerengemisches und die Lösung der restlichen Emulgatormenge und ein zweites Drittel des Starters gleichzeitig unter Abführung der Polymerisationswärme nach und nach einträgt und nach Abklingen der Polymerisations-Wärmeentwicklung durch Nachdosieren des letzten Drittels der Startermenge einen weitgehenden Umsatz der Monomeren sicherstellt.

2. Verfahren zur Herstellung einer Wasserstoffperoxidzubereitung nach Anspruch 1, dadurch gekennzeichnet, daß das Copolymerisat 0,02 bis 0,04 Gew.-% eines zweifach ungesättigten, copolymerisierbaren Monomeren enthält.

3. Verfahren zur Herstellung einer Wasserstoffperoxidzubereitung nach Anspruch 1 oder 2, dadurch gekennzeichnet, daß das Copolymerisat aus

| 55 Gew.-% | Ethylacrylat |
|---|---|
| 35 Gew.-% | Methycrylsäure |
| 10 Gew.-% | Acrylsäure und |
| 0,025 Gew.-% | Ethylenglycoldimethacrylat besteht. |

4. Verfahren zur Herstellung einer Wasserstoffperoxidzubereitung nach Ansprüchen 1 bis 3, gekennzeichnet durch einen pH-Wert von 2 - 5 und einen Gehalt von

| 1 - 20 Gew.-% | Wasserstoffperoxid und |
|---|---|
| 1 - 5 Gew.-% | (Feststoff) eines Copolymerisats einer Dispersion gemäß Ansprüchen 1 bis 3. |

5. Verfahren zur Herstellung einer Wasserstoffperoxidzubereitung nach Ansprüchen 1 bis 4, gekennzeichnet durch einen pH-Wert von mehr als 8.

6. Verwendung der Wasserstoffperoxidzubereitung nach Anspruch 5 als Entwickler für Oxidationsfärbemittel oder Haarbleichmittel.

**Claims**
**Claims for the following Contracting States : BE, CH, DE, FR, GB, IT, LI, NL, SE**

1. A hydrogen peroxide composition containing an aqueous dispersion of a copolymer having a solids content of from 5 to 30% by weight, based on the dispersion, said composition comprising

| 50-60% by weight of | ethyl acrylate, |
|---|---|
| 30-40% by weight of | methacrylic acid, |
| 5-15% by weight of | acrylic acid, and |
| 0.02- 0.04% by weight of | a poly-unsaturated copolymerizable monomer. |

2. The hydrogen peroxide composition according to claim 1, characterized in that said copolymer contains from 0.02 to 0.04% by weight of a double-unsaturated copolymerizable monomer.

3. The hydrogen peroxide composition according to claims 1 or 2, characterized in that said copolymer consists of

| 55% by weight of | ethyl acrylate, |
|---|---|
| 35% by weight of | methacrylic acid, |
| 10% by weight of | acrylic acid, and |
| 0.025% by weight of | ethyleneglycol dimethacrylate. |

4. The hydrogen peroxide composition according to claims 1 to 3, characterized by a pH value of from 2 to 5 and a content of

| 1-20% by weight of | hydrogen peroxide, and |
|---|---|
| 1- 5% by weight (solids) | of a copolymer of a dispersion according to claims 1 to 3. |

5. The hydrogen peroxide composition according to claims 1 to 4, characterized by a pH value of more than 8.

6. Use of the hydrogen peroxide composition according to claim 5 as developer for oxidation dyes or hair bleach.

**Claims for the following Contracting State : AT**

1. A process for preparing a hydrogen peroxide composition containing an aqueous dispersion of a copolymer having a solids content of from 5 to 30% by weight, based on the dispersion, said composition comprising

| 50-60% by weight of | ethyl acrylate, |
|---|---|
| 30-40% by weight of | methacrylic acid, |
| 5-15% by weight of | acrylic acid, and |
| 0.02- 0.04% by weight of | a poly-unsaturated copolymerizable monomer |

by the monomer addition process, wherein an aqueous solution containing about one third of the initiator and about 40% by weight of the emulsifiers is charged, and an atmosphere of inert gas is generated above the solution,

part of the monomer mixture is first introduced with stirring into the container warmed up to 70-80 °C, and after the reaction has started, the remainder of said monomer mixture and the solution of the residual amount of emulsifier and one second third of the initiator are simultaneously introduced by and by with a removal of polymerization heat,

and after the evolution of polymerization heat has quietened down, a high degree of conversion of the monomers is ensured by the subsequent addition of the last third of the amount of initiator.

2. The process for preparing a hydrogen peroxide composition according to claim 1, characterized in that said copolymer contains from 0.02 to 0.04% by weight of a double-unsaturated copolymerizable monomer.

3. The process for preparing a hydrogen peroxide composition according to claims 1 or 2, characterized in that said copolymer consists of

| 55% by weight of | ethyl acrylate, |
|---|---|
| 35% by weight of | methacrylic acid, |
| 10% by weight of | acrylic acid, and |
| 0.025% by weight of | ethyleneglycol dimethacrylate. |

4. The process for preparing a hydrogen peroxide composition according to claims 1 to 3, characterized by a pH value of from 2 to 5 and a content of
   1-20% by weight of hydrogen peroxide, and
   1- 5% by weight (solids) of a copolymer of a dispersion according to claims 1 to 3.

5. The process for preparing a hydrogen peroxide composition according to claims 1 to 4, characterized by a pH value of more than 8.

6. Use of the hydrogen peroxide composition according to claim 5 as developer for oxidation dyes or hair bleach.

**Revendications**
**Revendications pour les Etats contractants suivants : BE, CH, DE, FR, GB, IT, LI, NL, SE**

1. Composition de péroxyde d'hydrogène contenant une dispersion aqueuse d'un copolymère ayant une teneur en matière sèche de 5 à 30 % en poids, par rapport à la dispersion, composée de :
   - 50-60 % en poids d'acrylate d'éthyle
   - 30-40 % en poids d'acide méthacrylique
   - 5-15 % en poids d'acide acrylique et
   - 0,02-0,04 % en poids d'un monomère copolymérisable, plusieurs fois insaturé.

2. Composition de peroxyde d'hydrogène selon la revendication 1, caractérisée en ce que le copolymère contient de 0,02 à 0,04 % en poids d'un monomère copolymérisable, deux fois insaturé.

3. Composition de peroxyde d'hydrogène selon la revendication 1 ou 2, caractérisée en ce que le copolymère est composé de :
   - 55 % en poids d'acrylate d'éthyle
   - 35 % en poids d'acide méthacrylique
   - 10 % en poids d'acide acrylique et
   - 0,025 % en poids de diméthacrylate d'éthylèneglycol.

4. Composition de peroxyde d'hydrogène selon l'une quelconque des revendications 1 à 3, caractérisée par un pH de 2-5 et une teneur de :
   - 1-20 % en poids en peroxyde d'hydrogène et
   - 1- 5 % en poids (en matière sèche) en d'un copolymère d'une dispersion selon les revendications 1 à 3.

5. Composition de peroxyde d'hydrogène selon l'une quelconque des revendications 1 à 4, caractérisée par un pH de plus de 8.

6. Utilisation de la composition de peroxyde d'hydrogène selon la revendication 5, en tant que développeur pour produit de teinture par oxydation ou décolorant.

**Revendications pour l'Etat contractant suivant : AT**

1. Procédé pour la préparation d'une composition de peroxyde d'hydrogène contenant une dispersion aqueuse d'un copolymère ayant une teneur en matière sèche de 5-30 % en poids, composé de
   - 50-60 % en poids d'acrylate d'éthyle
   - 30-40 % en poids d'acide méthacrylique
   - 5-15 % en poids d'acide acrylique et
   - 0,02-0,04 % en poids d'un monomère copolymérisable plusieurs fois insaturé,
   par le procédé d'addition de monomère, dans lequel on dispose au préalable une solution aqueuse qui contient eviron un tiers de l'amorceur et environ 40 % en poids des émulsifiants, et on engendre sur la solution une atmosphère de gaz inerte, on introduit dans la charge préliminaire, chauffée aux environs de 70-80° C, sous agitation, d'abord une partie du mélange de monomères, et, après l'amorçage de la réaction, le reste du mélange de monomères, puis on introduit peu à peu simultanément la solution de la quantité restante d'émulsifiants et un second tiers de l'amorceur, en ~ minant la chaleur de polymérisation, et une fois cessé le dégagement de chaleur de polymérisation, on garantit une conversion poussée des monomères par post-addition réglée du dernier tiers de la quantité d'amorceur

2.  Procédé pour la préparation d'une composition de peroxyde d'hydrogène selon la revendication 1, caractérisé en ce que le copolymère contient de 0,02 à 0,04 % en poids d'un monomère copolymérisable, deux fois insaturé.

3.  Procédé pour la préparation d'une composition de peroxyde d'hydrogène selon la revendication 1 ou 2, caractérisé en ce que le copolymère est composé de :
    - 55 % en poids d'acrylate d'éthyle
    - 35 % en poids d'acide méthacrylique
    - 10 % en poids d'acide acrylique et
    - 0,025 % en poids de diméthacrylate d'éthylèneglycol

4.  Procédé pour la préparation d'une composition de peroxyde d'hydrogène selon l'une quelconque des revendications 1 à 3, caractérisé par un pH de 2-5 et une teneur de :
    - 1-20 % en poids en peroxyde d'hydrogène et
    - 1- 5 % en poids (en matière sèche) d'un copolymère d'une dispersion selon les revendications 1 à 3.

5.  Procédé pour la préparation d'une composition de peroxyde d'hydrogène selon l'une quelconque des revendications 1 à 4, caractérisé par un pH de plus de 8.

6.  Utilisation de la composition de peroxyde d'hydrogène selon la revendication 5, en tant que développeur pour produit de teinture par oxydation ou décolorant.